# EUROPEAN PATENT APPLICATION

(11) **EP 4 461 302 A1**
(43) Date of publication of application: **13.11.2024**
(21) Application number: 23740321.7
(22) Date of filing: 13.01.2023
(51) Int. Cl.: A61K 35/34, A61L 27/36, A61P 1/16, A61P 35/00, A61P 43/00

(54) **COMPOSITION FOR TREATING HEPATIC FUNCTION DISORDER**

(30) Priority: 14.01.2022 JP 2022004092
(71) Applicant: OSAKA UNIVERSITY, Suita-shi Osaka 565-0871 (JP); Terumo Kabushiki Kaisha, Tokyo 151-0072 (JP)
(72) Inventor: EGUCHI, Hidetoshi, Suita-shi, Osaka 565-0871 (JP); TOMIMARU, Yoshito, Suita-shi, Osaka 565-0871 (JP); MIYAGAWA, Shigeru, Suita-shi, Osaka 565-0871 (JP); TOYA, Keisuke, Suita-shi, Osaka 565-0871 (JP); OHASHI, Fumiya, Ashigarakami-gun, Kanagawa 259-0151 (JP)
(74) Representative: Prüfer & Partner mbB Patentanwälte · Rechtsanwälte
(86) International application number: PCT/JP2023/000699
(87) International publication number: WO 2023/136313

(57) **Abstract**

[Problem]

It is an object of the present invention to provide a means for treating liver dysfunction.

[Solution]

The above object was achieved by providing a composition containing skeletal myoblasts for treating liver dysfunction or improving liver function, in which the liver dysfunction can be hepatitis, liver fibrosis, liver cirrhosis, liver cancer, or liver failure; and the composition can be used for promoting the proliferation of liver cells, regeneration of the liver tissue and/or angiogenesis, and providing a method for treating liver dysfunction, including a step of treating applying the composition to a site exhibiting liver dysfunction.

## Description

### Technical Field

The present invention relates to a composition treating a liver dysfunction and others.

### Background Art

Recently, attempts have been made to implant various types of cells for repairing damaged tissues, etc. For example, for repairing myocardial tissue damaged by ischemic heart diseases such as angina pectoris and myocardial infarction, attempts have been made to use, e.g., fetal cardiomyocytes, skeletal myoblasts, mesenchymal stem cells, cardiac stem cells, ES cells and iPS cells (Non

### Patent Literature 1).

As part of the attempts, a cell construct formed on a scaffold and a sheet-shaped cell culture, which is a sheet formed of cells, have been developed (Patent Literature 1, Non Patent Literature 2).

For the application of the sheet-shaped cell culture to treatment, for example, studies have been made on use of an epidermal sheet-shaped cell culture for treating skin damages caused by, e.g., burn; use of a corneal epithelial sheet-shaped cell culture for corneal damage; and use of an oral mucosa sheet-shaped cell culture for esophageal cancer endoscopic resection. Some of them have entered the stage of clinical application.

As one of the applications, it has been proposed to use a sheet-shaped cell culture for treating damage to organs such as digestive tract. For example, Patent Literature 2 discloses the use of a sheet-shaped cell culture containing mesenchymal stem cells for treating or preventing leakage from a damaged portion of the gastrointestinal tract caused by suture failure, etc.

Furthermore, Patent Literature 3 discloses an engraftment sheet material having satisfactory engraftment efficiency to the surface of an organ and satisfactory operability in clinical applications. More specifically, it has been demonstrated that a myoblast sheet, which has an extracellular matrix layer on one of the surfaces and a biodegradable gel layer on the other surface, can be satisfactorily engrafted in the surface of the liver or colon by implanting the sheet such that the surface the extracellular matrix layer faces the organ.

### Citation List

### Patent Literature

Patent Literature 1: JP 2007-528755 A
Patent Literature 2: WO 2017/130802
Patent Literature 3: WO 2021/149830

### Non Patent Literature

Non Patent Literature 1: Haraguchi Y. et al., Stem Cells Transl. Med., 1(2), 136-141 (2012)
Non Patent Literature 2: Sawa Y. et al., Surg. Today, 42(2), 181-184 (2012)

### Summary of Invention

### Technical Problem

It is an object of the present invention to provide a means for treating liver dysfunction.

### Solution to Problem

The present inventors have studied a method for treating liver dysfunction. During the studies, they focused on the possibility of treating liver dysfunction by cytokines produced from cells. Then, they conducted studies with a view to demonstrating the possibility. As a result, they found that the proliferation of the liver cells, regeneration of the liver tissue and angiogenesis are promoted by cytokine produced from skeletal myoblasts. Based on the finding, they conducted further studies, with the result that the present invention was accomplished.

More specifically, the present invention relates to the followings.
[1] A composition containing skeletal myoblasts for treating liver dysfunction or improving liver function.
[2] The composition according to [1], in which the liver dysfunction is hepatitis, liver fibrosis, liver cirrhosis, liver cancer, or liver failure.
[3] The composition according to [1] or [2], in which the liver dysfunction is liver cirrhosis.
[4] The composition according to [1] or [2], in which the liver dysfunction is liver cancer.
[5] The composition according to [4], for application to the liver after liver cancer resection.
[6] The composition according to any one of [1] to [5], for promoting proliferation of liver cells, regeneration of the liver tissue and/or angiogenesis.
[7] The composition according to any one of [1] to [6], for treating liver dysfunction through cytokine production.
[8] A method for treating liver dysfunction, including a step of applying the composition according to any one of [1] to [7] to a site exhibiting liver dysfunction.
[9] The method according to [8], in which the step of applying the composition to a site exhibiting liver dysfunction is performed by an implantation device.
[10] The method according to [9], in which the implantation device is a catheter.

### Advantageous Effects of Invention

According to the present invention, it is possible to treat liver dysfunction such as hepatitis, liver fibrosis, liver cirrhosis, liver cancer, or liver failure. In particular, according to the present invention, it is possible to treat terminal-stage decompensated cirrhosis whose improvement cannot be expected by current internal therapy as well as to treat liver failure of residual liver after liver cancer resection. Furthermore, according to the present invention, it is possible to promote proliferation of the liver cells, regeneration of the liver tissue and/or angiogenesis. Moreover, according to the present invention, it is possible to treat liver dysfunction through cytokine production.

### Brief Description of Drawings

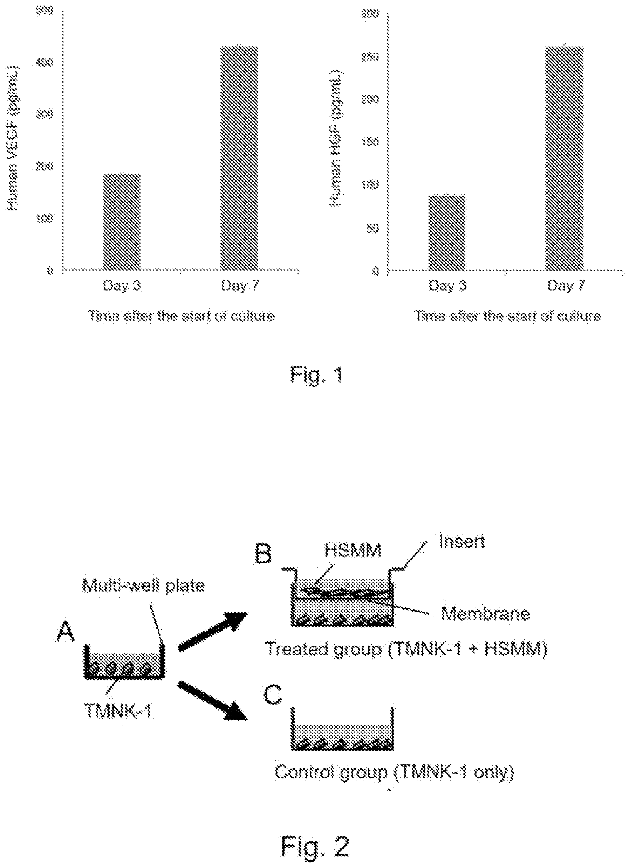

Fig. 1 shows the productions of growth factors derived from human skeletal muscle myoblasts. Each individual value is an average value (n = 3) and an error bar represents a standard deviation.
Fig. 2 shows the procedure of inducing the capillary formation of immortalized human liver endothelial cells (TMNK-1) by a factor derived from human skeletal muscle myoblasts (HSMM). HSMM (1.0 × 10⁵ cells)-containing medium soaked in a TMNK-1-containing medium (Fig. 2A) was designated as treated group 1 (n = 3) (Fig. 2B); HSMM (5.0 × 10⁵ cells)-containing medium soaked in a TMNK-1-containing medium (Fig. 2A) was designated as treated group 2 (n = 3) (Fig. 2B); and the medium not soaked was designated as a control group (n = 3) (Fig. 2C).

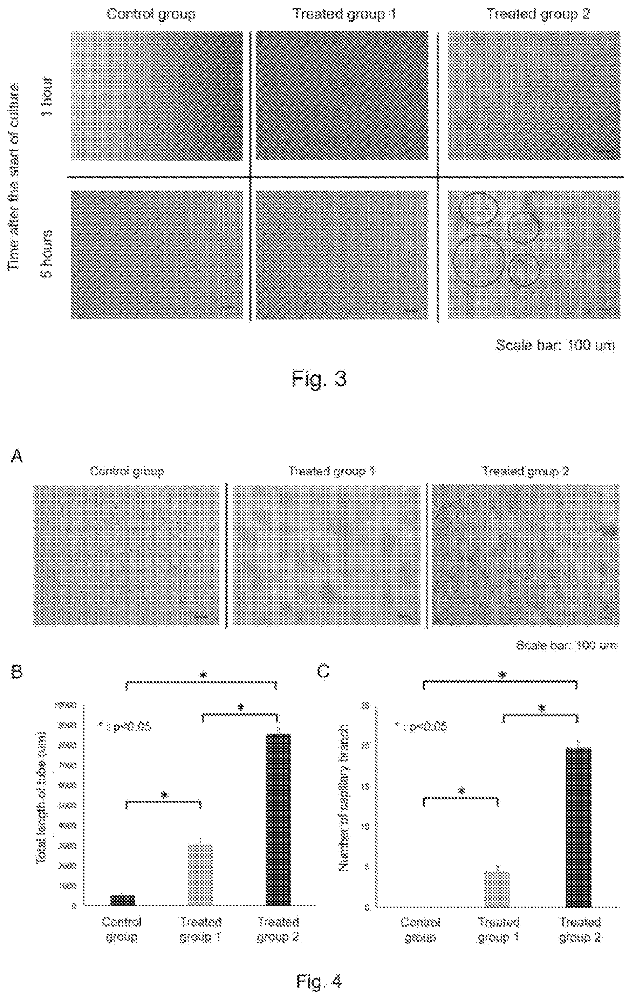

Fig. 3 shows optical microscope images (1 and 5 hours after the start of culture) of capillary formation of TMNK-1 induced by a factor derived from human skeletal muscle myoblasts. In the regions surrounded by a circle, capillary formation of TMNK-1 is induced by a factor derived from human skeletal muscle myoblasts.
Fig. 4 shows optical microscope images (24 hours after the start of culture) of capillary formation of TMNK-1 induced by a factor derived from human skeletal muscle myoblasts (Fig. 4A); the total length of a capillary observed in capillary formation of TMNK-1 induced by a factor derived from human skeletal muscle myoblasts (Fig. 4B), and the number of branches of the capillary (Fig. 4C). Each individual value is an average value of treated group 1 (n = 3), treated group 2 (n = 3), and control group (n = 3), respectively and an error bar represents a standard deviation.

In the specification, unless otherwise specified, all technical terms and scientific terms used in the specification have the same meaning that those skilled in the art commonly understand. All patents, applications and other publications and information cited herein are incorporated in their entirety herein by reference.

### Description of Embodiments

### [Composition for Treating Liver Dysfunction]

An aspect of the present invention relates to a composition containing skeletal myoblasts for treating liver dysfunction or improving liver function (sometimes referred to as "the composition of the present invention").

In an embodiment, liver dysfunction is hepatitis, liver fibrosis, liver cirrhosis, liver cancer, or liver failure.

In an embodiment, liver dysfunction is liver cirrhosis.

In an embodiment, liver dysfunction is liver cancer.

In an embodiment, the composition of the present invention is for application to the liver after liver cancer resection.

In an embodiment, the composition of the present invention is used for promoting the proliferation of liver cells, regeneration of the liver tissue and/or angiogenesis.

In an embodiment, the composition of the present invention is used for treating liver dysfunction through cytokine production.

The liver is an organ having a wide variety of functions such as metabolism, maintenance of body-fluid homeostasis, and digestion as well as regeneration ability. In the liver, blood flows in from the hepatic artery and portal vein, passes through the central vein and flows out from the hepatic vein. The liver tissue is constituted of a mass of liver lobules and the central vein passes through the central shaft part of a liver lobule. The liver cells are radially arranged around the central vein to form the liver cell plate and occupy most of the whole cells in the liver. In the specification, the "liver cells" are interchangeably used with "hepatic parenchymal cells". The cells other than the liver cells in the liver are called "liver non-parenchymal cells". Examples thereof include sinusoidal endothelial cells, Kupffer cells, dendritic cells, natural killer (NK) cells, hepatic stellate cells, and monocyte-derived macrophages.

In the present invention, the liver is not limited as long as it is the liver of a living organism. Examples of the liver include the liver of a human, a non-human primate, a rodent (such as a mouse, a rat, hamster or a guinea pig) and a mammal such as a rabbit, a dog, a cat, a pig, a horse, a cow, a goat or sheep.

In the present invention, the "liver function" refers to a function of a healthy liver such as metabolism, maintenance of body-fluid homeostasis and digestion.

In the present invention, the "liver dysfunction" refers to inhibition, suppression and/or impairment of the liver function due to some cause. For example, the liver dysfunction refers to hepatitis, liver fibrosis, liver cirrhosis, liver cancer, or liver failure.

In the present invention, the "healthy liver" refers to an unharmed (intact) liver not influenced by, e.g., a disease, a disorder and/or a treatment.

In the present invention, the term "skeletal myoblasts" refers to myoblasts present in the skeletal muscle. The skeletal myoblasts are commonly known in the technical field and can be prepared from the skeletal muscle by any one of the methods commonly known (for example, a method disclosed in JP 2007-89442) or is commercially available (for example, Lonza, Cat# CC-2580). Skeletal myoblasts may be identified by a maker. Examples of the marker include, but are not limited to, CD56, α7 integrin, myosin heavy chain IIa, myosin heavy chain IIb, myosin heavy chain IId (IIx), MyoD, Myf5, Myf6, myogenin, desmin and PAX3. In a specific embodiment, skeletal myoblasts are CD56 positive. Further, in a specific embodiment, skeletal myoblasts are CD56 positive and desmin positive. Skeletal myoblasts may be derived from any organism having skeletal muscle. Examples of the organism include, but are not limited to, a human, a non-human primate, a rodent (such as a mouse, a rat, a hamster or a guinea pig) and a mammal such as a rabbit, a dog, a cat, a pig, a horse, a cow, a goat or sheep. In an embodiment, the skeletal myoblasts refer to mammalian skeletal myoblasts. In a specific embodiment, the skeletal myoblasts refer to human skeletal myoblasts. Furthermore, the skeletal myoblasts can be collected from any skeletal muscle. In an embodiment, skeletal myoblasts refer to skeletal myoblasts derived from the thigh, neck or abdomen.

In the present invention, the "composition containing skeletal myoblasts" refers to a composition containing at least skeletal myoblasts and an additional component having biocompatibility. Herein, the additional component having biocompatibility refers to a component having no undesirable effect, such as an inflammatory response, an immune response or a toxic reaction, on living body tissues and cells, or having such an effect but at least the effect is low. Examples of the additional component include, but are not particularly limited to, a pharmaceutical acceptable carrier, an excipient, cells other than skeletal myoblasts, a component enhancing, e.g., viability, engraftment and/or functionality of the composition of the present invention, a component useful for reproduction and/or healing of living body tissues or promotion thereof, and/or a graft. Those skilled in the art familiar with the additional components and can appropriately select an additional component commonly known and use it. Furthermore, the aforementioned additional components can be used in combination with the composition of the present invention.

The composition containing skeletal myoblasts, although the state thereof is not particularly limited, exhibits, for example, a liquid-state having flowability, such as an aqueous solution, a suspension or an emulsion or a solid state having flexibility such as a sheet-shaped cell culture. Particularly, in view of application to the liver surface, the composition containing skeletal myoblasts is preferably a sheet-shaped cell culture. In an embodiment, the composition containing skeletal myoblasts is a pharmaceutical composition.

In the present invention, the "sheet-shaped cell culture" refers to a sheet formed of cells connected to each other. Cells may be mutually connected directly (including cells connected via a cellular element such as an adhesion molecule) and/or via an intervening substance. The intervening substance is not particularly limited as long as it can connect cells at least physically (mechanically). Examples thereof include an extracellular matrix. The intervening substance is preferably a cell-derived substance and particularly a substance derived from cells constituting a cell culture. Cells are at least physically (mechanically) connected and further may functionally, for example, chemically or electrically connected. The sheet-shaped cell culture may be composed of a single cell layer (uni-layer), two or more cell layers (a laminate (multilayer), for example, 2 layers, 3 layers, 4 layers, 5 layers or 6 layers). Furthermore, the sheet-shaped cell culture may not have a clear layer-structure of cells but has a three-dimensional structure having a thickness beyond the thickness of a single cell. For example, cells may not be uniformly arranged in the horizontal direction, that is, may be non-uniformly arranged in the vertical section of a sheet-shaped cell culture.

In an embodiment, the sheet-shaped cell culture of the present invention is extremely fragile. It is sometimes difficult to handle it. Accordingly, the sheet-shaped cell culture of the present invention may further have a reinforcement layer for the purpose of making handling easier and reducing a risk of breakage. The reinforcement layer is not limited as long as it does not impair the function of the sheet-shaped cell culture of the present invention and reinforces the structure thereof. The reinforcement layer may contain, for example, a gel and/or a polymer. However, since the sheet-shaped cell culture of the present invention is to be implanted into a living body, the reinforcement layer preferably a biocompatible reinforcement layer containing, e.g., a biocompatible gel or polymer.

The gel to be used in the reinforcement layer, preferably the biocompatible gel, is not limited as long as it does not adversely affect a living body when introduced. Examples of the gel include, but are not limited to, fibrin gel, fibrinogen gel, gelatin gel and collagen gel.

The polymer to be used in the reinforcement layer, preferably the biocompatible polymer, is not limited as long as it does not adversely affect a living body when introduced. Examples of the polymer include, but are not limited to, polylactic acid, polydioxano, polyglycapro and collagen.

In a case where a sheet-shaped cell culture having a reinforcement layer is applied to an application site, it is preferable that the sheet is applied such that the reinforcement layer is not directly in contact with the site. In other words, the sheet-shaped cell culture is preferably applied such that the sheet-shaped cell culture is positioned between an application site and the reinforcement layer.

The composition of the present invention is applied to the liver to treat liver dysfunction or improve liver function. In an embodiment, the composition of the present invention is applied to the liver surface to treat liver dysfunction or improve liver function. In an embodiment, the composition of the present invention is applied to the liver by an implantation device to treat liver dysfunction or improve liver function. In an embodiment, the composition of the present invention is applied to the surface of the liver by an implantation device to treat liver dysfunction or improve liver function.

In the present invention, the "surface of the liver" refers to any surface of a healthy liver or liver influenced in some way by, e.g., a disease, a disorder, and/or a treatment. In an embodiment, the liver surface is the surface of the residual liver after partial hepatectomy (for example, liver cancer resection). In an embodiment, the liver surface is the surface (having a fibrosis region) of the liver exhibiting liver fibrosis.

In the present invention, the "application" refers to using the composition of the present invention to a target site, for example, adding, administrating, injecting, delivering, applying, attaching, engrafting, and/or implanting the composition of the present invention to a target site.

In the present invention, the "implantation device" refers to a medical device enabling the composition of the present invention to apply to the liver. In an embodiment, the implantation device refers to a medical device enabling the composition of the present invention to apply to the liver surface. In a case where the composition of the present invention is a sheet-shaped cell culture, the implantation device is, for example, a medical device having a planar structure capable of supporting and detaching the sheet-shaped cell culture. In view of operating it within the abdominal cavity, the implantation device preferably has a form that can be inserted and passed through a cylindrical body which has been inserted in a body cavity in endoscopic surgery.

In the present invention, "hepatitis" refers to an inflammation in the liver. Examples of hepatitis include viral hepatitis (such as hepatitis A, B and C), alcoholic hepatitis, nonalcoholic steatohepatitis, drug-induced hepatitis and autoimmune hepatitis. Hepatitis is roughly divided into acute hepatitis and chronic hepatitis. Acute hepatitis is hepatitis that temporally occurs, whereas chronic hepatitis is hepatitis lasting for a long time, with the result that, e.g., a fibrotic change of the liver, and degeneration and necrosis of liver cells, may occur. Chronic hepatitis develops, through, e.g., a fibrotic change of the liver and degeneration and necrosis of liver cells, into liver fibrosis and liver cirrhosis, and then, the liver cancer.

In the present invention, "liver fibrosis" refers to the formation of a scar tissue in the liver, which is developed by replacing the parenchymal tissue of a healthy liver for connective tissue by a fibrotic change of the liver and degeneration and necrosis of liver cells caused by repetitive damage and chronic hepatitis, followed by accumulating an extracellular matrix component such as collagen.

In the present invention, "liver cirrhosis" refers to the formation of a large amount of scar tissue in the liver, which is developed by replacing the parenchymal tissue of a healthy liver for a large amount of connective tissue by a fibrotic change of the liver, and degeneration and necrosis of liver cells caused by repetitive damage and chronic hepatitis, followed by accumulating a large amount of an extracellular matrix component such as collagen. Liver cirrhosis in the initial stage having no symptoms is called compensated cirrhosis (a failure caused in part of the liver can be compensated by the remaining part). In contrast, liver cirrhosis in the terminal stage having a symptom such as jaundice, spider hemangioma and palmar erythema, which appears as a disease state progresses, is called decompensated cirrhosis. Compensated cirrhosis and initial-stage decompensated cirrhosis can be treated by current internal therapy (e.g., interferon, glycyrrhizin preparation, diuretic, albumin preparation and diet remedy). In contrast, in the terminal-stage decompensated cirrhosis, no improvement thereof is expected by current internal therapy. After the disease progresses into liver cancer and liver failure, there is no choice but a surgical treatment.

In an embodiment, the composition of the present invention is used for treating compensated cirrhosis or decompensated cirrhosis. In an embodiment, the composition of the present invention is used for treating initial- or terminal-stage compensated cirrhosis or decompensated cirrhosis.

In the present invention, "liver cancer" refers to a malignant tumor arising in the liver. Liver cancer is roughly divided into primary liver cancer and metastatic liver cancer. The primary liver cancer is mostly hepatocellular carcinoma (malignant tumor derived from liver cells). Hepatocellular carcinoma often results from progression to liver cancer through chronic hepatitis, liver fibrosis, liver cirrhosis.

In the present invention, "liver failure" refers to a significant reduction in the function of healthy liver, such as metabolism, body-fluid homeostasis and digestion, due to damage to most of the liver. Examples of the cause for liver failure include viral hepatitis, liver cirrhosis, alcoholic liver injury and drug-induced liver injury. Furthermore, the residual liver after partial hepatectomy (for example, after liver cancer resection) sometimes shows liver failure. Liver failure is roughly classified into acute liver failure and chronic liver failure. Acute liver failure quickly progresses in a short period of time, whereas chronic liver failure gradually progresses over a long period of time.

In the present invention, the phrase "applied to the liver after liver cancer resection" refers to applying the composition of the present invention to the residual liver after liver cancer is partially or completely removed. In an embodiment, the composition of the present invention is applied to the surface of the residual liver after liver cancer is partially or completely removed. In an embodiment, the composition of the present invention is applied by an implantation device to the surface of the residual liver after liver cancer is partially or completely removed. In an embodiment, the liver after liver cancer resection shows liver failure.

In the present invention, "liver cells" refer to cells radially arranged around the central vein of a liver lobule in the liver to form the liver cell plate and made up of a majority of the whole cells in the liver. In the specification, the "liver cells" are interchangeably used with "hepatic parenchymal cells".

In the present invention, "liver tissue" refers to a tissue constituted of a mass of liver lobules.

In the present invention, "angiogenesis" refers to a phenomenon where new blood vessels are formed from preexisting vessels in, e.g., the liver and branched to construct a vascular network.

In the present invention, the "cytokine production" refers to producing cytokines such as vascular endothelial growth factor (VEGF) and hepatocyte growth factor (HGF), which play a role in, e.g., angiogenesis, protection and repairment of cells. For example, in the liver to which the composition of the present invention was applied, cytokines such as VEGF and HGF, which play a role in, e.g., angiogenesis, protection and repairment of cells, are produced.

The composition of the present invention can contain optional cells in addition to skeletal myoblasts. The optional cells are not particularly limited and may include for example, adhesion cells (adherent cells). Examples of the adhesion cells include, adhesive somatic cells (for example, cardiomyocytes, fibroblasts, epithelial cells, endothelial cells, liver cells, pancreatic cells, kidney cells, adrenal gland cells, periodontal ligament cells, gingival cells, periosteal cells, skin cells, synovial cells, cartilage cells) and stem cells (for example, tissue stem cells such as myoblasts, cardiac stem cells, pluripotent stem cells such as embryonic stem cells, iPS (induced pluripotent stem) cells and mesenchymal stem cells). The somatic cells may be differentiated from stem cells, particularly iPS cells (iPS cell-derived adherent cells). Non-limiting examples of cells include myoblasts, mesenchymal stem cells (for example, cells derived from bone marrow, fat tissue, peripheral blood, skin, hair root, muscle tissue, endometrium, placenta, umbilical cord blood), cardiomyocytes, fibroblasts, cardiac stem cells, embryonic stem cells, iPS cells, synovial cells, cartilage cells, epithelial cells (for example, oral mucosal epithelial cells, retinal pigment epithelial cells, nasal mucosal epithelial cells), endothelial cells (for example, vascular endothelial cells), liver cells (for example, hepatic parenchymal cells), pancreatic cells (for example, pancreatic islet cells), kidney cells, adrenal gland cells, periodontal ligament cells, gingival cells, periosteal cells, and skin cells. Non-limiting examples of the iPS cell-derived adherent cells include iPS cell-derived cardiomyocytes, fibroblasts, epithelial cells, endothelial cells, liver cells, pancreatic cells, kidney cells, adrenal gland cells, periodontal ligament cells, gingival cells, periosteal cells, skin cells, synovial cells, and cartilage cells.

The cells in the composition of the present invention may be derived from an organism which can be treated with the composition of the present invention. Examples of the organism include, but are not limited to, a human, a non-human primate, a dog, a cat, a pig, a horse, a goat, sheep, a rodent (for example, a mouse, a rat, a hamster, a guinea pig) and a rabbit. Furthermore, the number of types of cells in the composition of the present invention is not particularly limited. The composition may be constituted of only one type of cell or two types or more of cells. In a case where the number of types of cells in the composition is two or more, the content ratio (purity) of the most common type of cell is 50% or more, preferably 60% or more, more preferably 70% or more, and further preferably 75% or more, at the time when completion of the formation of the composition of the present invention.

In an embodiment, the cells are autogenic cells. In a case where the composition of the present invention is used for implantation, the "autogenic cells" herein refer to cells derived from a recipient.

In an embodiment, the cells are isogenic cells. Herein, the "isogenic cells" refer to cells derived from the same species as the recipient and similar species based on the genetical relatedness (in other words, no rejection reaction occurs after implantation to the recipient). For example, in a case where the recipient is a human, human cells of the similar species based on the genetical relatedness correspond to isogenic cells.

In an embodiment, the cells are allogeneic cells. Herein, the "allogeneic cells" refer to cells derived from an organism belonging to the same species as the recipient but different species based on the genetical relatedness (in other words, a rejection reaction occurs after implantation to the recipient). For example, in a case where the recipient is a human, human cells of the different species based on the genetical relatedness correspond to allogeneic cells.

In the specification, the "autogenic cells" are used interchangeably with "autogenic cells", and "isogenic cells" and "allogeneic cells" are interchangeably used with "xenogenic cells".

In an embodiment, cells are heterogenic cells. In a case where the composition of the present invention is used for implantation, the "heterogenic cells" herein refer to cells derived from an organism of different species from the recipient. For example, in a case where the recipient is a human, cells derived from a monkey or a pig correspond to the heterogenic cells.

The autogenic cells and isogenic cells are preferable in the present invention because no rejection reaction occurs even if they are implanted. However, it is possible to use allogeneic cells and heterogenic cells. In a case where the allogeneic cells and heterogenic cells are used, an immunosuppressive treatment is often required to suppress a rejection reaction.

In a case where skeletal myoblasts are prepared from the striated muscle tissue, fibroblasts are contained in the cell population thus prepared. In a case where the composition of the present invention is produced using a cell population containing skeletal myoblasts prepared from the striated muscle tissue, the cell population contains a predetermined amount of fibroblasts. Fibroblasts are well known in the technical field and can be identified by a marker such as TE-7 (see, for example, Rosendaal et al., J Cell Sci. 1994; 107 (Pt 1): 29-37, Goodpaster et al., J Histochem Cytochem. 2008; 56 (4): 347-58).

In an embodiment, cells in the composition of the present invention include skeletal myoblasts prepared from striated muscle tissue. Accordingly, the cell population to be used in producing the composition of the present invention may contain skeletal myoblasts and fibroblasts. In an embodiment, the cell population to be used in producing the composition of the present invention has a CD56 positive rate of 50% or more, 60% or more, 65% or more, 70% or more, 75% or more, 80% or more, 85% or more or 90% or more and preferably 60% or more.

The cell population to be used in producing the composition of the present invention may contain fibroblasts. However, in a case where the content rate of fibroblasts is too high is not preferable since the content rate of the skeletal myoblasts decreases. Therefore, in one embodiment, the cell population used in the production of the composition of the present invention may have a TE7 positive rate of 50% or less, 40% or less, 35% or less, 30% or less, 25% or less, 20% or less, 15% or less, or 10% or less, and preferably 40% or less.

The cell population to be used in producing the composition of the present invention may contain cells other than skeletal myoblasts and fibroblasts but the content of the other cells is preferably as low as possible. Accordingly, the higher the total value of the CD56 positive rate and the TE7 positive rate, the more preferable. The total value is, for example, 80% or more, 85% or more, 90% or more, 95% or more, 96% or more, 97% or more, 98% or more or 99% or more and preferably 90% or more.

In an embodiment, the composition of the present invention may be applied to a tissue, for example, in combination with another composition and/or graft for promoting healing. Examples of the composition and/or graft for promoting healing include, but are not limited to, a graft containing a vascular pedicle such as an omentum majus piece, fibrin gel and Adspray (registered trademark). In a preferred embodiment, the composition of the present invention is applied together with a graft containing a vascular pedicle. A representative example of the graft containing a vascular pedicle is an omentum majus piece.

The composition and/or graft for promoting healing may be a composition independent of the composition of the present invention, or may be integrated into the composition of the present invention.

In a case where the composition of the present invention is applied in combination with another composition and/or a graft for promoting heating independently of the composition of the present invention, the composition and/or graft may be applied before or after the application of the composition of the present invention. In a case where the composition and/or graft is applied before the application of the composition of the present invention, the composition and/or graft is applied so as to be positioned between an application site and the composition of the present invention. More specifically, the composition and/or graft is first applied to an application site, and thereafter, the composition of the present invention is applied over the composition and/or graft. In a case where the composition and/or graft is applied after the application of the composition of the present invention, the composition and/or graft is applied to an application site with the composition of the present invention interposed between them. More specifically, the composition of the present invention is first applied to an application site, and then, the composition and/or graft is applied on the composition of the present invention.

A mechanism for tissue regeneration is considered, for example, due to a paracrine effect, which is bought by sustained-release of the composition of the present invention of e.g., cytokines such as VEGF, HGF and collagen, which play a role in, e.g., angiogenesis, and protection and repair of cells at an affected site; and/or, due to an autocrine effect, which is brought by e.g., collagen production promoted by activation of progenitor cells or stem cells of the tissue around the application site.

In a case where the composition of the present invention takes the form of a sheet-shaped cell culture, the size of the sheet-shaped cell culture of the present invention is not particularly limited as long as it can cover a predetermined part of living tissue. If the sheet-shaped cell culture is, for example, circular, the diameter thereof is 10 to 55 mm, 15 to 50 mm, 20 to 45 mm, 25 to 40 mm, or 30 to 35 mm.

### [Method for Treating Liver Dysfunction]

Another aspect of the present invention relates to a method for treating liver dysfunction, including a step of applying the composition of the present invention to a site exhibiting liver dysfunction (sometimes referred to as "the treatment method of the present invention").

In the present invention, "site exhibiting liver dysfunction" refers to a liver site at which liver function is impaired or suppressed and/or a liver site that lost liver function. In an embodiment, the site exhibiting liver dysfunction is a site of the liver surface at which liver function is impaired or suppressed and/or a site of the liver surface losing liver function. In an embodiment, the site exhibiting liver dysfunction is a site of the liver surface exhibiting hepatitis, liver fibrosis, liver cirrhosis, liver cancer, or liver failure. In an embodiment, the site exhibiting liver dysfunction is a site of the liver surface exhibiting compensated cirrhosis or decompensated cirrhosis. In an embodiment, the site exhibiting liver dysfunction is a site of the liver surface exhibiting initial- or terminal-stage decompensated cirrhosis In an embodiment, the site exhibiting liver dysfunction is a site of the surface of residual liver exhibiting liver failure after liver cancer resection.

In an embodiment, in the treatment method of the present invention, the step of applying the composition of the present invention to a site exhibiting liver dysfunction is performed by an implantation device. In an embodiment, the implantation device refers to a medical device enabling the composition of the present invention to apply to the liver surface. In a case where the composition of the present invention is a sheet-shaped cell culture, the implantation device is, for example, a medical device having a planar structure capable of supporting and detaching the sheet-shaped cell culture. In view of operating it within the abdominal cavity, the implantation device preferably has a form that can be inserted and passed through a cylindrical body which has been inserted in a body cavity in endoscopic surgery. In an embodiment, the implantation device is a catheter.

In the present invention, the "catheter" refers to a medical tube having flexibility. The composition of the present invention can be applied to a site exhibiting liver dysfunction by inserting a catheter to, e.g., an abdominal cavity or a blood vessel. The composition of the present invention can be added, administered, injected, delivered, applied, attached, engrafted, and/or implanted to a site exhibiting liver dysfunction by inserting a catheter to, e.g., an abdominal cavity and a blood vessel.

In an embodiment, in the treatment method of the present invention the step of applying the composition of the present invention to a site exhibiting liver dysfunction is a step of adding, administering, injecting delivering, applying, attaching, engrafting, and/or implanting the composition of the present invention to a site exhibiting liver dysfunction.

### [Use in Producing Medicine for Treating Liver Dysfunction]

Another aspect of the present invention relates to use of the composition of the present invention in producing a medicine (sometimes referred to as "use in producing the medicine of the invention") for treating liver dysfunction or improving liver function.

### [Use for Treating Liver Dysfunction]

Another aspect of the present invention relates to use of the composition of the present invention, (sometimes referred to as "use of the present invention") for treating liver dysfunction or improving liver function.

### [Method for Promoting Proliferation and Others of Liver Cells]

Another aspect of the present invention relates to a method for promoting proliferation of liver cells, regeneration of the liver tissue and/or angiogenesis, including a step of co-culturing the composition of the present invention with liver cells, liver tissue, and/or endothelial cells (sometimes referred to as "the method of the present invention for promoting e.g., proliferation of liver cells). The method of the present invention for promoting e.g., proliferation of liver cells, promotes proliferation of liver cells, regeneration of the liver tissue and/or angiogenesis through cytokine production.

In the present invention, the "endothelial cells" are cells constituting the endothelium in which capillary formation is likely induced by a factor derived from skeletal myoblasts. In an embodiment, the endothelial cells are vascular endothelial cells constituting artery, vein, and/or capillaries. In an embodiment, the endothelial cells are liver endothelial cells. In one embodiment, the endothelial cells are sinusoidal endothelial cells. In one embodiment, the endothelial cells are the sinusoidal endothelial cells of the liver.

In the present invention, the "co-culture" refers to culturing a plurality of cells and/or a plurality of types of cells in the same environment.

In an embodiment, the method of the present invention for promoting, e.g., proliferation of liver cells is performed *in vitro.*

In an embodiment, the method of the present invention for promoting, e.g., proliferation of liver cells is performed, *in vivo.*

The present invention will be more specifically described with reference to the following Examples, which show specific examples of the present invention and would not limit the present invention.

### Examples

### Example 1. Production of growth factors Derived from human skeletal muscle myoblasts

To a culture container (IWAKI tissue-culture dish (surface treated for adherent cells) (3020-100), AGC TECHNO GLASS Co., Ltd.), Dulbecco's modified eagle medium (DMEM) (Nacalai Tesque Inc.) was added. Subsequently, 1.0 × 10⁶ of human skeletal muscle myoblasts (HSMM) (Normal HSMM, Lonza K.K.) were seeded. Culture was performed at 37°C, in the condition of 5% CO₂. The culture medium after 3 days from the start of culture was centrifuged at 2,000 × g for 10 minutes, and the supernatant was collected (n = 3). The supernatant was subjected to quantification based on ELISA using a commercially available ELISA kit (Human VEGF ELISA Kit (ab222510) and Human HGF ELISA Kit (ab100534), Abcam) for a vascular endothelial growth factor (VEGF) and a hepatocyte growth factor (HGF). Furthermore, HSMM was cultured in the same manner as above and the supernatant of 7 days after the start of culture was collected (n = 3) and subjected to quantification based on ELISA for each of VEGF and HGF. For the color reaction in ELISA, the optical density (OD) at 450 nm was measured by a microplate reader (680 XR, Bio-Rad Laboratories, Inc.).

As a result, it was confirmed that the expression level of each of VEGF and HGF increases from 3 to 7 days after the start of culture (Fig. 1). VEGF is known to produce HGF from sinusoidal endothelial cells through the VEGFR2-ID1 signal transduction system in the sinusoidal endothelial cells of the liver. Furthermore, HGF is known to promote the proliferation of liver cells and the reproduction of liver tissue. Thus, it was found that HSMM promotes the proliferation of liver cells and the reproduction of liver tissue through cytokine production such as VEGF and HGF.

### Example 2. Induction of Capillary Formation by Factor Derived from Human Skeletal Muscle Myoblasts

### (1) Preparation of Immortalized Human Liver Endothelial Cells (TMNK-1)-containing Medium

To a Transwell (registered trademark) multiwell plate (6.5 mm, attached with 0.4 um Pore Polyester Membrane Insert, Corning), Dulbecco's modified eagle medium (DMEM) (Nacalai Tesque Inc.) was added, and subsequently, 1.0 × 10⁵ of immortalized human liver endothelial cells (TMNK-1) (given by JCRB cell bank, cell number (JCRB): JCRB1564) were seeded (TMNK-1-containing medium) (Fig. 2A).

### (2) Preparation of Human Skeletal Myoblast (HSMM)-containing Medium

To a Transwell (registered trademark) insert, DMEM was added and subsequently, 1.0 × 10⁵ of human skeletal myoblasts (HSMM) (Normal HSMM, Lonza K.K.) were seeded (HSMM (1.0 × 10⁵ cells)-containing medium). Separately, to a Transwell (registered trademark) insert, DMEM was added and subsequently, 5.0 × 10⁵ of HSMM were seeded (HSMM (5.0 × 10⁵ cells)-containing medium).

### (3) Soaking HSMM-containing Medium in TMNK-1-containing Medium

The Transwell (registered trademark) insert, which contains HSMM (1.0 × 10⁵ cells)-containing medium and was prepared in the above (2), was inserted in the Transwell (registered trademark) multiwell plate, which contains TMNK-1-containing medium and was prepared in the above (1). In this manner, the HSMM-containing medium was soaked in the TMNK-1-containing medium and designated as treated group 1 (n = 3) (Fig. 2B). Similarly, the Transwell (registered trademark) insert, which contains HSMM (5.0 × 10⁵ cells)-containing medium and was prepared in the above (2), was inserted in the Transwell (registered trademark) multiwell plate, which contains TMNK-1-containing medium and was prepared in the above (1). In this manner, the HSMM-containing medium was soaked in the TMNK-1-containing medium and designated as treated group 2 (n = 3) (Fig. 2B). The soaking made it possible to exchange various factors between the HSMM-containing medium and TMNK-1-containing medium through the membrane of the Transwell (registered trademark) insert. A Transwell (registered trademark) multiwell plate, which contains the TMNK-1-containing medium, was prepared in the above (1) and not soaked, was used as a control group (n = 3) (Fig. 2C).

### (4) Induction of Capillary Formation

Treated group 1, treated group 2, and the control group described in the above (3) were cultured at 37°C in the condition of 5% CO₂. One hour, 5 hours and 24 hours after the start of culture, the Transwell (registered trademark) insert of each of treated group 1 and treated group 2 was removed. TMNK-1 contained in each of Transwell (registered trademark) multiwell plates of the treated group 1, treated group 2, and the control group was observed by an optical microscope at a 200-fold magnification.

As a result, it was confirmed that TMNK-1 capillary formation was induced by HSMM, 5 hours after the start of culture in treated group 2 compared to the control group (Fig. 3). Furthermore, it was confirmed that TMNK-1 capillary formation was induced by HSMM, 24 hours after the start of culture in treated groups 1 and 2 compared to the control group (Fig. 4A). Moreover, it was confirmed that the total length of the capillary in treated group 2 is longer and the number of branches thereof are larger than treated group 1, 24 hours after the start of culture (Figs. 4B and C). More specifically, it was confirmed that the larger the number of HSMM cells in the HSMM-containing medium, the more actively the capillary formation of TMNK-1 is induced by HSMM. Thus, it was found that TMNK-1 capillary formation is induced by the factor derived from HSMM; in other words, angiogenesis is promoted.

From the results in the foregoing, according to the present invention, liver dysfunction such as hepatitis, liver fibrosis, liver cirrhosis, liver cancer, or liver failure can be treated. In particular, according to the present invention, it is possible to treat terminal-stage decompensated cirrhosis whose improvement cannot be expected by current internal therapy as well as to treat liver failure of residual liver after liver cancer resection. Furthermore, according to the present invention, it is possible to promote proliferation of the liver cells, regeneration of the liver tissue and/or angiogenesis. Moreover, according to the present invention, it is possible to treat liver dysfunction through cytokine production.

## Claims

1. A composition comprising skeletal myoblasts for treating liver dysfunction or improving liver function.

2. The composition according to claim 1, wherein the liver dysfunction is hepatitis, liver fibrosis, liver cirrhosis, liver cancer, or liver failure.

3. The composition according to claim 1 or 2, wherein the liver dysfunction is liver cirrhosis.

4. The composition of claim 1 or 2, wherein the liver dysfunction is liver cancer.

5. The composition according to claim 4, for application to the liver after resection of liver cancer.

6. The composition according to any one of claims 1 to 5, for promoting proliferation of liver cells, regeneration of a liver tissue and/or angiogenesis.

7. The composition according to any one of claims 1 to 6, for treating liver dysfunction through cytokine production.

8. A method for treating liver dysfunction, the method comprising a step of applying the composition according to any one of claims 1 to 7 to a site exhibiting liver dysfunction.

9. The method according to claim 8, wherein the step of applying the composition to a site exhibiting liver dysfunction is performed by an implantation device.

10. The method according to claim 9, wherein the implantation device is a catheter.
